# EUROPEAN PATENT SPECIFICATION

(11) **EP 4 180 067 B1**
(45) Date of publication and mention of the grant of the patent: **03.01.2024**
(21) Application number: 21207588.1
(22) Date of filing: 10.11.2021
(51) Int. Cl.: A61M 1/16, A61M 1/34

(54) **DEVICE FOR EXTRACORPOREAL BLOOD TREATMENT**
VORRICHTUNG ZUR EXTRAKORPORALEN BLUTBEHANDLUNG
APPAREIL DE TRAITEMENT SANGUIN EXTRACORPOREL

(43) Date of publication of application: 17.05.2023
(73) Proprietor: Ritter, Kai-Uwe, 34212 Melsungen (DE)
(72) Inventor: Ritter, Kai-Uwe, 34212 Melsungen (DE)
(74) Representative: Ziebig Hengelhaupt Intellectual Property Attorneys Patentanwaltskanzlei PartGmbB

(56) References cited:
- EP-A1- 2 666 491
- EP-A1- 2 883 566
- EP-A1- 3 903 846
- WO-A1-2020/011784
- US-A1- 2006 254 982

## Description

### FIELD OF THE INVENTION

The invention relates to a dialysis machine comprising an extracorporeal blood conduction system and a dialyzing fluid system, wherein the extracorporeal blood conduction system is in communication with the dialyzing fluid system via at least one dialyzer and further comprises at least one blood pump which can convey fluid in the extracorporeal blood conduction system, wherein the extracorporeal blood conduction system comprises at least one venous and one arterial conduit, the arterial conduit leading up to the dialyzer and the venous conduit leading away from the dialyzer and the respective other end of the venous and arterial conduit being intended to be connected to a patient, further comprising at least one substitute conduit for supplying fresh substitution fluid either into the arterial conduit or into the venous conduit or into both the arterial and the venous conduit, characterized in that at least two hydraulic connections are present at the dialysis machine, to each of which one substitute conduit is connectable for supplying fresh substitution fluid, wherein at least two further hydraulic connections are present, wherein the at least two further hydraulic connections are waste ports that, in use, convey used fluid to the drain.

### BACKGROUND OF THE INVENTION

The medical treatment of patients with acute and chronic renal insufficiency in special clinics or in hospitals places high demands on personnel and material-technical requirements. Outpatient and inpatient dialysis facilities are among the areas where patients and staff are at particular risk of infection.

As a result of the reduced cellular defenses caused by chronic uremia, in particular dialysis patients and renal patients are more susceptible to certain infections. Depending on the dialysis procedure, haemodialysis (HD), haemodiafiltration (HDF) or peritoneal dialysis (PD), patients are exposed to different infection risks.

The risk of transmitting infectious diseases (e.g. viral hepatitis B or C), the high technical treatment costs and the need to repeat dialysis treatment regularly are the main reasons why dialysis treatment requires a high level of hygiene awareness from all who are involved.

For instance, according to Section 23, Paragraph 5 of the German Infection Protection Act, dialysis facility managers are required and obligated to define internal procedures for an infection hygiene plan. The hygiene plan must be accessible and visible to all employees at all times and must be regularly checked to ensure that it is up to date and brought to the attention of employees. The participation in the training sessions is mandatory for all employees and must be documented.

The risk of infection is generally determined by the presence of primary pathogens, the transmission routes of the pathogens (direct or indirect, natural or artificial routes of transmission) and the patient's defense and immune situation. Due to the above-mentioned specific patients, dialysis facilities are exposed to numerous infection risks. These include a higher prevalence of multi-resistant bacterial pathogens (MRE).

With regard to the dialysis machines, it must be ensured that those (including those used in home dialysis) are operated according to the manufacturer's instructions, disinfected, cleaned and maintained according to the manufacturer's instructions. In particular, the disinfection of the dialysis machine includes the hydraulic circuit and the surface. Dialysis machines are disinfected after each use on the patient and irrespective of the presence of an infection.

For disinfection of the hydraulic circuit, only the validated disinfection procedures approved by the respective device manufacturer are allowed to be used. Afterwards the hydraulic circuit is tested to ensure that there are no residues of disinfectant. The most frequently used disinfectant for chemical disinfection is usually citric acid 50% in combination with boiling or nearly boiling water (hot citric thermal disinfection). This combination advantageously also removes calcification from the machine hydraulics. Another disinfectant method is applying only boiling or nearly boiling water in order to kill any germs by means of heat, this is called thermal disinfection.

However, within dialysis machines capable of performing haemodiafiltration, the sterile substitution fluid is provided at a port, which is dedicated merely for this purpose. Said port can be closed for disinfection in such a way that the disinfectant flows through and around said port during disinfection of the hydraulic circuit. Furthermore, when said port is closed, the fluid is returned via the closure, which encloses the port. In particular if a dialysis machine capable of performing online HDF, wherein fresh dialysis fluid is processed with one or multiple filtration steps and infused as substitution fluid, said substitution port must always be sterile in order to prevent any germs to enter into the patient. After being used in one therapy, this port has to be considered contaminated.

Although, strictly speaking, there is only this one critical point on the dialysis machine that can get contaminated between two therapies, the entire hydraulic system is disinfected on all machines nowadays. Due to the long paths in the hydraulics, limited heating power, pre- and post-rinsing time, as well as a necessary exposure time, the disinfection step takes approx. 30-45 minutes, which is why some countries do not disinfect between two successive therapies.

If a therapy is given with HD only, the risk of contamination is very low because the dialysis fluid in single-pass is used only once and always goes from the dialysis fluid inlet of the machine to the dialysis fluid outlet of the machine, which is connected to the drain. Accordingly, inside the machine, the fresh dialysis fluid does not get into contact with the potentially contaminated used dialysis fluid.

However, if a therapy is performed with HDF or HD with PBR, additional sterile filtered dialysis fluid, i.e. substitution fluid, flows from the substitute port directly into the bloodstream. This fluid is also used to fill the blood tubes before treatment, so called "Priming". It is also used to provide a fluid bolus during treatment, if this is required. Furthermore, substitution fluid is used to return the blood after the end of the therapy. This is done in an instance by connecting the arterial side of the bloodline to the substitution port. The blood is then returned venously with the substitution fluid using the blood pump. To avoid direct contamination of the port with blood, an intermediate piece can be connected to the arterial line, which is sterile. Another method is to connect the arterial line to an additional connection on the conduit downstream of the substitution port, to supply substitution fluid to the bloodline, this way avoiding the disconnection of the conduit from the substitution port. The blood is then returned venously with the substitution fluid using the blood pump. This is called rinseback. Accordingly, PBR stands for priming, bolus and rinseback.

However, despite all measures, there is a risk that the port may be contaminated by touching at connection or disconnection of the conduit, or forgetting the intermediate piece during rinseback. It is therefore necessary, and for the HDF/PBR devices used nowadays even mandatory by the manufacturer's instructions, to disinfect between therapies.

### DESCRIPTION OF THE RELATED ART

Within the state-of-the-art dialysis machines having two hydraulic connectors to which a respective connection line having a filter coupling is connected for connection to a dialyzer are known, according to US 2020/0384175 A1. However, the hydraulic connectors being an inflow connector for feeding fresh dialysis fluid to the dialyzer and a backflow connector for draining used dialysis fluid from the dialyzer, and having two parking positions associated with the respective hydraulic connectors for storing the filter couplings when not in operation. Thus, this prior art, by no means, discloses the use of two hydraulic connectors for subsequent usage in order to reduce the required disinfection times. Additionally, WO 2012/010322 A2 discloses a connector suitable to be emptied by means of sterile air before being connected to a functional device, so that the fluid guides and contact surfaces of the connector provided for fluid connection are cleaned of possible germs. However, any dialysis machine comprising said connector still requires an entire disinfection circle between two treatments Further prior art is found in EP2666491 A1, EP2883566 A1, US2006254982 A1, WO2020011784 A1 and EP3909846 A1.

Usually, two to three shifts are run with one dialysis machine within a dialysis center per day. This means one break between two therapies, or two breaks for three therapies. However, none of the prior art addresses the need for avoidance of disinfection of the substitution port in order to reduce the time required between two or more consecutive treatments.

### SUMMARY OF THE INVENTION

Based on the prior art described above, the present invention is based on the object of eliminating the disadvantages mentioned above, in particular to provide a device for extracorporeal blood treatment which requires less time for disinfection purposes. Phrased differently, the objective of the present invention is to provide a dialysis machine that reduces the number of cleaning and disinfection procedures, specifically that does not require hydraulics disinfection between at least two consecutive treatments. In other words, the number of cleaning or disinfection procedures per use of the dialysis machine has to be reduced. More specifically this applies to dialysis machines, that produce sterile, or at least ultra-pure substitution fluid for the purpose of priming, bolus and rinse back (PBR) and/or additionally to perform haemodiafiltration or hemofiltration treatments.

Accordingly, the invention is intended to reduce treatment costs and simplify the treatment process. Since all parts of a dialysis machine that get into contact with the patient's blood, like the dialyzer, the arterial and the venous conduits, are replaced after each treatment, and the reinfusion often occurs via the substitution solution, wherein the arterial line is connected to the flushing connection of the substitution line between the substitution port and the substitution pump, merely the disinfection of the substitution port and the waste port is required in order to prepare the dialysis machine for the next treatment. All other relevant conduits or ports which might provide a gateway or and entry point for germs and viruses are replaced.

HDF can be performed with different techniques. The classic HDF technique used an average reinfusion rate of 9 liters/session in the post-dilution mode. A blood flow over 300 ml/min was required for sufficient rates of dialysis fluid at acceptable transmembrane pressure (TMP) gradients. The equipment included an dialysis fluid control system, a reinfusion pump and a scale to weigh reinfusion bags. Another variant of HDF, called 'high-volume HDF', used 15 liters or more of reinfusion per session. Online HDF (OL-HDF) is used to prepare large amounts of inexpensive replacement solution, and HDF can be performed with a very high fluid turnover (up to 25-30 liters/session). Substitution fluid can be infused in either pre- or post-dilution mode, or both, in different proportions.

Within the description several terms are used interchangeably, for instance the terms *"substitution fluid"* and *"substitute fluid",* wherein both terms are used for defining the fluid that represent the substitute fluid within a dialysis machine. Accordingly, the terms *"substitute conduit"* and *"substitution conduit*" both refer to the line or tube in which the fluid that represent the substitute fluid within a dialysis machine is transferred, moved, pumped etc. Furthermore, the dialysis fluid and its conduit system are referred to as *"dialysis fluid inlet*" and *"dialysis fluid outlet*", respectively. Accordingly, fresh dialysis fluid is pumped into the dialyzer at the inlet, whereas the used dialysis fluid returns at the *"dialysis fluid outlet*" or *"dialysis fluid drain",* which are both used interchangeably, respectively. Where used, the terms *"inleY"* and *"outlet"* also define the direction of fluid flow within the system, meaning the fluid flows from the inlet to the outlet. Accordingly, any fluid that flows towards the waste port flows out of the dialyzing fluid system.

According to the invention, an object is achieved by a dialysis machine, comprising an extracorporeal blood conduction system and a dialyzing fluid system, wherein the extracorporeal blood conduction system is in communication with the dialyzing fluid system via at least one dialyzer, further comprising at least one blood pump which can convey fluid in the extracorporeal blood conduction system, wherein the extracorporeal blood conduction system comprises at least one venous and one arterial conduit, the arterial conduit leading up to the dialyzer and the venous conduit leading away from the dialyzer and the respective other end of the venous and arterial conduit being intended to be connected to a patient, further comprising at least one substitute conduit for supplying fresh substitution fluid either into the arterial conduit or into the venous conduit or into both the arterial and the venous conduit, characterized in that at least two hydraulic connections are present at the dialysis machine, to each of which one substitute conduit is connectable for supplying fresh substitution fluid, wherein at least two further hydraulic connections are present, wherein the at least two further hydraulic connections are waste ports that, in use, convey used fluid to the drain.

According to a preferred embodiment, said hydraulic connections are substitution ports. The at least two substitution ports are never accessible at the same time. In other words, at any time only one of the at least two substitution ports is accessible for connecting with a substitution conduit, while the at least one other substitution port is locked and/or not available for connection.

The at least two substitution ports are arranged at the machine's front face of the extracorporeal blood treatment machine/dialysis machine which allows to be easily and quickly accessible by the user.

According to another preferred embodiment the dialysis machine further comprises at least one substitution pump which can convey dialysis fluid in each of the at least two substitute conduits, respectively.

Advantageously, this allows volumetric supplying of substitution fluid. The main purpose of the substitution pump is to control the amount of substitution fluid delivered during priming, bolus, rinse-back or haemodiafiltration, in particular the inlet of the pump is connected to the supply of substitution fluid after filtration, while the outlet, downstream of the pump connects pre- and/or post-dialyzer to the extracorporeal blood conduction system, i.e., the arterial conduit or into the venous conduit or into both conduits.

According to another preferred embodiment of the present invention, the substitution ports are each equipped with a connection sensor.

This ensures in an advantageous manner that the two hydraulic connections, i.e. the substitution ports are recognized, for example by providing each of the hydraulic connections with a separate cover in each case. Accordingly, only one hydraulic connection is available for treatment with one patient at a time. Phrased differently, the connection ports are covered with a lid or similar means while not in use, in order to maintain their clean status after disinfection and avoid contamination while not being used, or to avoid use, when the lid is locked, in case the port is used or machine is in disinfection.

Further, according to another embodiment the at least two hydraulic connections are each provided with a respective status indicator.

This enables the operator in an advantageous and easy manner to clearly determine which of the hydraulic ports has already been in use and thereby is considered contaminated. Additionally, this prevents incorrect connection and allows the operator to quickly, easily and unambiguously determine which of the at least two hydraulic ports have already been used for the at least one previous treatment.

In one embodiment, such a status indicator is identified by a red and green light, for example, a red and green LED, or OLED, wherein a red light identifies the hydraulic connection that has already been used and the green light identifies the hydraulic connection that has not yet been used. A blue light indicates flow from the port during use. The indicators can be used to signal to the user when to connect or disconnect conduits to a certain connection port by a certain color or by flashing.

This advantageously supports ensuring that only one hydraulic connection is ready for being used for treatment with one patient at a time.

Further, according to a different embodiment the at least two hydraulic connections are Luer connectors, or nozzles onto which an adapter can be plugged.

This allows interoperability of the provided machine with the available standard equipment and enables the respective user to readily apply and use the machine without further excessive instructions, or the need for new, additional material which would create additional costs and waste - with regard to the unused material of the previously used machine, which has not been used up.

According to another preferred embodiment of the present invention the dialyzer is a high-flux hemodialyzer with a membrane having an ultrafiltration coefficient >20 ml/h per 1 mm Hg per 1 m² and a beta₂-microglobulin clearance of >=20ml/Min.

This allows the improved removal of middle molecular weight uremic toxins, reduce intradialytic hypotension, and reduce dialysis related pathology, such as amyloidosis and accelerated atherosclerosis during a dialysis treatment of a patient.

Membranes differ in their clearance capacities of different solutes basically depending on thickness and pore size. However, increasing the pore size and reducing thickness is almost forcedly associated to a water permeability increase. The permeability of a membrane to water, generally expressed in milliliters per hour per millimeter of mercury is determined according to ANSI/AAMI/ISO 8637:2010. A lox-flux hemodialyzer used in the present invention has a membrane with an ultrafiltration coefficient <=10 ml/h per 1 mm Hg per 1 m² and a beta₂-microglobulin clearance of <=10ml/Min.

Further, according to another embodiment of the invention, the convection volume of at least 20% of the total blood volume processed is delivered, while the fluid balance is maintained.

This supports the further improved removal of middle molecular weight uremic toxins.

According to another preferred embodiment of the present invention, the dialysis machine comprises at least two further hydraulic connections for the removal of used dialysis fluid.

These hydraulic connections for the removal of used dialysis fluid are called waste ports which are used during priming and during rinse-back to receive excess fluid. This supports the reduction of the number of cleaning or disinfection procedures per use of the dialysis machine since once the waste port is used one time, it is considered contaminated and has to be disinfected before the next treatment, whereby the provision of two or more of those connections reduces the need for disinfection.

According to another embodiment of the present invention the waste ports can be paired with the respective substitution ports, i.e. port-pairs, which also share at least one cover per pair.

This allows further securitizing that double use of those ports is prevented. Phrased differently, where a hydraulic connection for substitution fluid supply and a hydraulic connection for the removal of used fluid, i.e. substitution port and waste port, are combine to a port-pair, they can share one cover per pair.

Additionally with regard to a further preferred embodiment of the present invention, the port-pairs are each equipped with a connection sensor, or a sensor for detection of an opened and/or closed lid.

This advantageously ensures that the port-pairs are recognized, for example by providing each of the pairs with a separate cover in each case. Accordingly, only one port-pair is used for treatment with one patient at a time. Phrased differently, the connection ports are covered with a lid or similar means while not in use, in order to maintain their sterile status after disinfection and avoid contamination while not being used, or to avoid use, when the lid is locked, since the port has already been used or machine is in disinfection procedure.

According to an even more preferred embodiment of the invention, the at least two port-pairs are each provided with a respective status indicator.

The status indicator enables the operator to clearly determine which of the port-pairs has already been in use and thereby is considered contaminated. This prevents incorrect connection and allows the operator to quickly, easily and unambiguously determine which of the at least two port-pairs has already been used for the at least one previous treatment.

In one embodiment, such a status indicator is identified by a red and green light, for example, a red and green LED, or OLED, where a red light identifies the port-pair or port-pairs that has or have already been used and the green light identifies the port-pair or port-pairs that has or have not yet been used. A blue light indicates flow from the port-pair during use.

This advantageously supports ensuring that only one port-pair is used for treatment with one patient at a time. The indicators signal to the user when to connect or disconnect conduits to a certain connection port by a certain color or by flashing.

Furthermore, preferably three substitution ports and three waste ports are present. Even more preferably four substitution ports and four waste ports are present. Most preferably five substitution ports and five waste ports are present.

This allows the subsequent treatment of four, five or even six patients without requiring disinfection between the treatments.

According to another embodiment, the dialysis machine comprises at least one filtration device.

This allows the filtration of the dialysis fluid in order to remove bacteria and Endotoxins, and thus create sterile, or at least ultrapure fresh dialysis fluid.

In particular, the microbiological limits (total aerobic count in CFU/ml, endotoxins in IU/ml) of the dialysis fluid are:
Bacteria detection < 100 CFU/ml
Endotoxin detection < 0,25 IU/ml

In the case of substitution solution prepared online by the dialysis machine, the socalled sterile filter is supplemented by one or more online filters, wherein the microbiological limits for online substitution fluid are:
Total microbial count: < 10⁻⁶ CFU/ml
Endotoxin detection: < 0,03 IU/ml

Within another embodiment, the at least one blood pump and/or the at least one substitute pump, comprised by the dialysis machine according to the present invention, is a peristaltic pump.

This allows for continuous operation with correct selection of size, speed, pressure range, wherein the peristaltic pump is a completely closed system with smooth surfaces, and thus easy to sterilize in order to ensure no contamination of the pumped material by lubricants of the pump and gentle conveyance of blood cells, which would be destroyed by rapidly rotating propeller blades, as well as precise metering of very small flow rates possible with minimal maintenance required.

Within another embodiment of the present invention, a substitution port and a waste port are grouped together in order to form a port-pair and wherein at least two port-pairs are equipped with separate covers, wherein the connection sensors detect an opening of one of the covers, while a control mechanism locks the cover of the remaining hydraulic connections. Accordingly, the port-pairs are equipped with separate covers, wherein the connection sensors detect an opening of one of the covers, while a control mechanism locks the cover of the remaining port-pairs. Preferably the blocking occurs via electromagnetic or electromechanical means. Thereby blocking the at least second hydraulic connection, or second port-pair, avoids contamination thereof. In addition, the locking avoids unintentional opening of the connections during disinfection, whereby the risk of hot acidic fluid spilling to the user is reduced.

Within another embodiment of the present invention, the control mechanism further controls the status of the hydraulic connections and the dialysis machine.

This allows an inclusion of the control of the hydraulic connections within the machine control software, which enhances the overall operational safety and reduces the need for additional safeguard mechanisms, for instance for leakage control.

According to another embodiment of the present invention, all hydraulic ports are included within the hydraulic disinfection process.

This enhances the performance of the invention, since the initial aim of reducing the amounts of cleaning and disinfection procedures between two or more treatments, since the cleaning and disinfection of all ports at once is achieved by the above-mentioned feature. Advantageously, the disinfection process occurs via thermal disinfection.

In one embodiment of the present invention, the dialysis machine further comprises at least one valve which controls the flow of fresh substitution fluid from each of the hydraulic connections or of used substitution fluid to the hydraulic connections, wherein the at least one valve is controlled by the control mechanism.

The respective at least one valve remains closed while a substitution port and/or a waste port is/are not being used, especially in case substitution port and/or a waste port has/have been used, to avoid migration of contamination into the hydraulic circuit. During disinfection the valves will be open to allow effective disinfection of the substitution ports and/or waste ports.

It is clear that more than two blood pumps and/or substitution pumps, like 3, 4, 5 or even 6 pumps, respectively, could be present and within the scope of the present invention.

### EXAMPLES

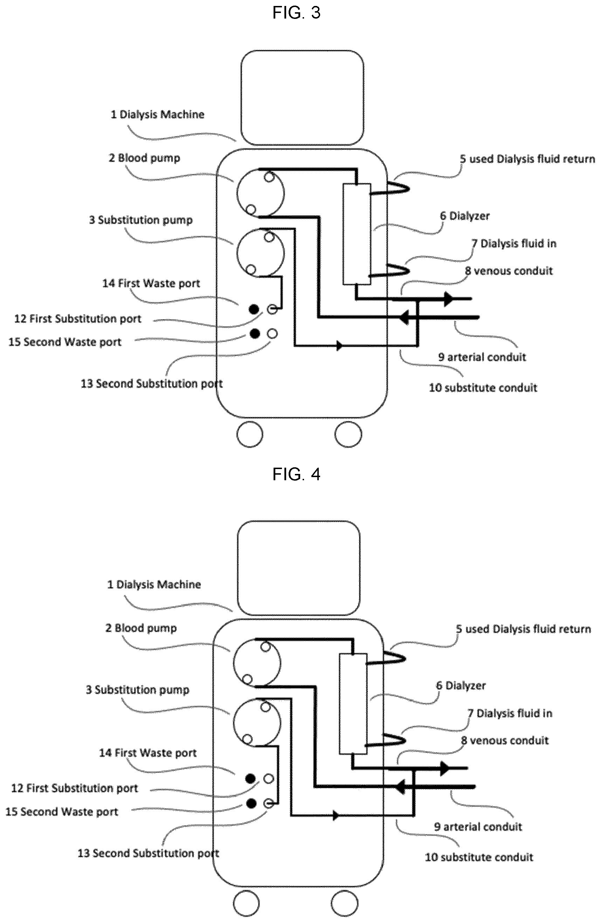

### 1. HDF machine in post-dilution mode with 2 sets of ports (Fig 3 and Fig. 4)

As an example, the setup of an HDF dialysis machine according to the present invention is explained in a step-by-step fashion:

### Treatment of a 1^{st} patient. (Fig.3)

The dialysis machine hydraulic has been disinfected before use, including the substitution and waste port connections (12,13,14,15).

After the concentrates and the bicarbonate solutions have been connected, the arterial and venous conduit systems are inserted, wherein the respective blood conduits are guided through corresponding clamps and connected to the respective sensors (not shown). The conduit 10 is connected from the venous blood conduit (8) downstream of the dialyzer to the 1^{st} substitution port (12), which is indicated by a green LED (not shown), and whose lid is unlocked by the control system (not shown) and the conduit 10 is inserted in the substitution pump (3).

For priming, the patient end of the venous line is connected to the 1^{st} waste port (14) which is indicated by a green LED (not shown), and whose lid is unlocked by the control system (not shown).

Substitution fluid from the 1^{st} substitution port (12) is pumped by the substitution pump (3) and is used to prime the conduit system and purge the air from it, wherein the blood pump (2) and substitution pump (3) are automatically stopped by the system as soon as the predetermined filling level is reached. The air and excess fluid flows to the 1^{st} waste port (14) and from there to the drain.

After the respective treatment parameters like dialysis fluid composition, blood flow, dialysis fluid flow, HDF substitution fluid volume or rate, minimum and maximum parameters, and heparin, if required, are entered, the dialyzer is coupled to the dialyzing fluid system. The system is now tested for leaks within the dialyzing fluid system. Afterwards, the dialysis machine is ready for the 1^{st} patient to be connected for treatment.

The 1^{st} patient is connected to the blood conduits. The lid of the 1^{st} waste port (14) is closed and locked (not shown). A valve allowing fluid to flow from the 1^{st} substitution port (12) is opened (not shown) and the blood pump (2) and the substitution pump (3) are started, dialysis fluid flow is started - Treatment is started and typically lasts 3.5-4h.

### Fluid bolus during treatment

During treatment, a fluid bolus can be provided. The blood pump (2) and the substitution pump (3) will be stopped. Now the substitution pump (3) will be started again to administer a certain amount of fluid, for instance 200 ml. The fluid is flowing from the 1^{st} substitution port (12) through the substitution pump (3) to the venous blood conduit (8) and to the patient's venous connection. Once the required amount is administered, the substitution pump (3) is stopped. The therapy will continue.

### End of 1^{st} Therapy

At the end of the therapy the blood pump (2) and the substitution pump (3) are stopped. The blood reconstitution can happen in two steps. In a first step, the valve for substitution fluid from the 1^{st} substitution fluid port (12) is opened (not shown). The substitution pump (3) will pump substitution fluid to the venous blood conduit (8) and push back the blood in the venous blood conduit (8) to the patient. Once this is finished, the venous blood conduit (8) is clamped. In a 2^{nd} step, the blood pump (2) will be started in reverse direction, the substitution pump (3) will be started at the same speed, thus providing fluid form the 1^{st} substitution port (12) to push back the blood in the arterial blood conduit (9) to the patient. Once this is finished, the blood pump and the substitution pump will stop and the arterial blood conduit (9) is clamped proximal to the patient. Now all the blood is returned to the patient and the patient can be disconnected from the arterial (9) and venous (8) blood conduit.

The blood conduits and the dialyzer can now be removed from the dialysis machine. The lid of the 1^{st} substitution port (12) and 1^{st} waste port (14) will be closed and then locked by the system, the LEDs for the used 1^{st} set of ports will turn red (not shown). The surface of the machine will be disinfected.

End of 1^{st} Therapy, machine is ready for the 2^{nd} treatment. A hydraulic disinfection is not required.

### Treatment of a 2^{nd} patient. (Fig.4)

The user indicates on the user interface of the machine that a new (2^{nd}) treatment shall be started.

The system will unlock the lids for the 2^{nd} set of ports and switch the LEDs for the 2^{nd} set of ports from red to green to indicate readiness for the setup of the 2^{nd} treatment.

After the concentrates and the bicarbonate solutions have been connected, the arterial and venous conduit systems are inserted, wherein the respective blood conduits are guided through corresponding clamps and connected to the respective sensors. The conduit 10 is connected from the venous blood conduit (8) downstream of the dialyzer to the 2^{nd} substitution port (13), which is indicated by a green LED (not shown), and whose lid is unlocked by the control system (not shown) and the conduit 10 is inserted in the substitution pump (3).

For priming, the patient end of the venous line is connected to the 2^{nd} waste port (15) which is indicated by a green LED (not shown), and whose lid is unlocked by the control system (not shown).

Substitution fluid from the 2^{nd} substitution port (13) is pumped by the substitution pump (3) and is used to prime the conduit system and purge the air from it, wherein the blood pump (2) and substitution pump (3) are automatically stopped by the system as soon as the predetermined filling level is reached. The air and excess fluid flows to the 2^{nd} waste port (15) and from there to the drain.

After the respective treatment parameters like dialysis fluid composition, blood flow, dialysis fluid flow, HDF substitution fluid volume or rate, minimum and maximum parameters, and heparin, if required, are entered, the dialyzer is coupled to the dialyzing fluid system. The system is now tested for leaks within the dialyzing fluid system. Afterwards, the dialysis machine is ready for the 2^{nd} patient to be connected for treatment.

The 2^{nd} patient is connected to the blood conduits. The lid of the 2^{nd} waste port (15) is closed and locked (not shown). A valve (not shown) allowing fluid to flow from the 2^{nd} substitution port (13) is opened. and the blood pump (2) and the substitution pump (3) are started, dialysis fluid flow is started. Treatment is started and typically lasts 3.5-4h.

### Fluid bolus during treatment

During treatment, a fluid bolus can be provided. The blood pump (2) and the substitution pump (3) will be stopped. Now the substitution pump (3) will be started again to administer a certain amount of fluid, for instance 200 ml. The fluid is flowing from the 2^{nd} substitution port (13) through the substitution pump (3) to the venous blood conduit (8) and to the patient's venous connection. Once the required amount is administered, the substitution pump (3) is stopped. The therapy will continue.

### End of 2^{nd} Therapy

At the end of the therapy the blood pump (2) and the substitution pump (3) are stopped. The blood reconstitution can happen in two steps. In a first step, the valve for substitution fluid from the 2^{nd} substitution fluid port (13) is opened. The substitution pump (3) will pump substitution fluid to the venous blood conduit (8) and push back the blood in the venous blood conduit (8) to the patient. Once this is finished, the venous blood conduit (8) is clamped. In a 2^{nd} step, the blood pump (2) will be started in reverse direction, the substitution pump (3) will be started at the same speed, thus providing fluid form the 2^{nd} substitution port (13) to push back the blood in the arterial blood conduit (9) back to the patient. Once this is finished, the blood pump and the substitution pump will stop, and the arterial blood conduit (9) is clamped proximal to the patient. Now all the blood is returned to the patient and the patient can be disconnected from the arterial (9) and venous (8) blood conduit

The blood conduits, and the dialyzer can now be removed from the dialysis machine. The lid of the 2^{nd} substitution port (13) and 2^{nd} waste port (15) will be closed and then locked by the system, the LEDs for the used 2^{nd} set of ports will turn red (not shown). The surface of the machine will be disinfected.

End of 2^{nd} Therapy.
Both port pairs have been used, the machine needs to be disinfected before any next treatment. The lids of the ports cannot be unlocked and opened before the disinfection has been performed successfully

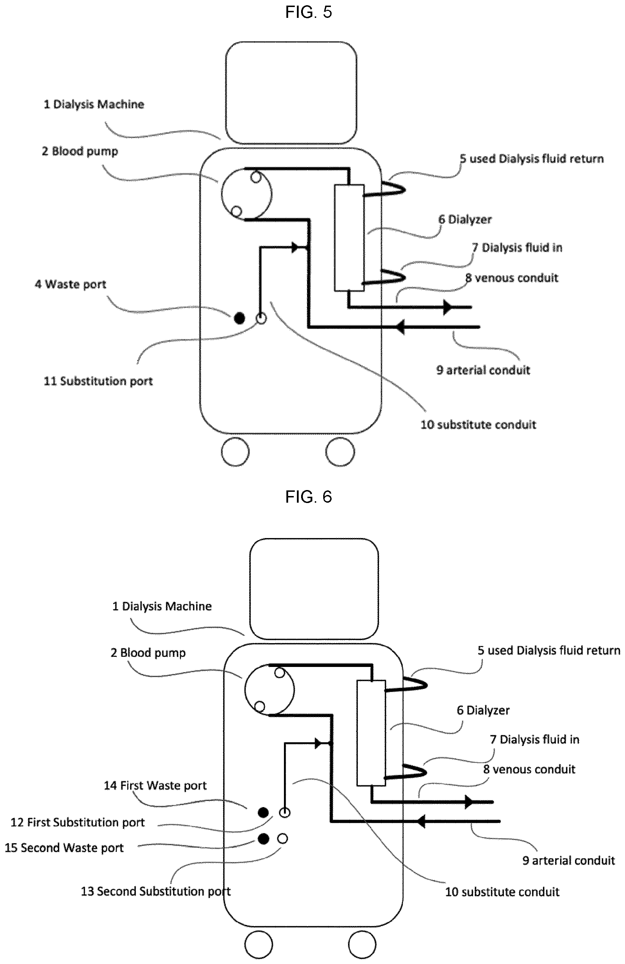

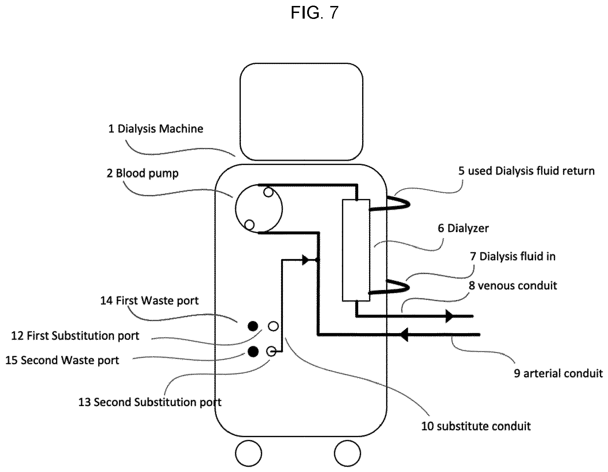

### 2. PBR machine with 2 sets of ports (Fig 6 and figure7)

As an example, the setup of a PBR dialysis machine according to the present invention is explained in a step-by-step fashion:

### Treatment of a 1^{st} patient. (Fig.6)

The dialysis machine hydraulic has been disinfected before use, including the substitution and waste port connections (12,13,14,15).

After the concentrates and the bicarbonate solutions have been connected, the arterial and venous conduit systems are inserted, wherein the respective blood conduits are guided through corresponding clamps and connected to the respective sensors. The conduit (10) is connected pre-pump between the arterial blood conduit (9) to the 1^{st} substitution port (12), which is indicated by a green LED, and whose lid is unlocked by the control system (not shown).

For priming, the patient end of the venous line is connected to the 1^{st} waste port (14) which is indicated by a green LED, and whose lid is unlocked by the control system (not shown).

Substitution fluid from the 1^{st} substitution port (12) is used to prime the conduit system and purge the air from it, wherein the blood pump stops the system automatically as soon as the predetermined filling level is reached. The air and excess fluid flows to the 1^{st} waste port (14) and from there to the drain.

After the respective treatment parameters like dialysis fluid composition, blood flow, dialysis fluid flow, minimum and maximum parameters, and heparin, if required, are entered, the dialyzer is coupled to the dialyzing fluid system. The system is now tested for leaks within the dialyzing fluid system. Afterwards, the dialysis machine is ready for the 1^{st} patient to be connected for treatment.

The 1^{st} patient is connected to the blood conduits. The lid of the waste port is closed and locked and the blood pump is started, dialysis fluid flow is started. Treatment is started and typically lasts 3.5-4h.

### Fluid bolus during treatment

During treatment, a fluid bolus can be provided. The blood pump will be stopped, then a valve will be opened to allow flow from the 1^{st} substitution port (12) to the blood conduit, the arterial line will be clamped upstream from the point where the substitution fluid enters the blood conduit. Now the blood pump will be started to administer a certain amount of fluid, for instance 200 ml. As the arterial line is clamped, the fluid is flowing from the 1^{st} substitution port (12) to the blood conduit and then is being pumped to the patient's venous connection. Once the required amount is administered, the fluid valve will be closed to stop flow from the 1^{st} substitution port (12) and the arterial conduit can be unclamped. The therapy will continue.

### End of 1^{st} Therapy

At the end of the therapy the blood pump is stopped. The blood reconstitution can happen in two steps. In a first step, the valve for substitution fluid from the 1^{st} substitution fluid port (12) is opened. As there is a positive pressure from the 1^{st} substitution port (12), the substitution fluid will flow to the blood conduit and push back the blood in the arterial blood conduit to the patient. Once this is finished, the arterial blood conduit is clamped proximal to the patient. In a 2^{nd} step, the blood pump will be started, thus using fluid form the 1^{st} substitution port (12) to push back the blood into the venous blood conduit to the patient. Once this is finished, the blood pump will stop and the venous blood conduit is clamped proximal to the patient. Now all the blood is returned to the patient and the patient can be disconnected from the arterial and venous blood conduit.

The blood conduit, and the dialyzer can now be removed from the dialysis machine. The lid of the 1^{st} substitution port (12) and 1^{st} waste port (14) will be closed and then locked, the LEDs for the used 1^{st} set of ports will turn red (not shown).

The surface of the machine will be disinfected.

End of 1^{st} Therapy, machine is ready for the 2^{nd} treatment. A hydraulic disinfection is not required.

### Treatment of the 2^{nd} patient (Fig 7)

The user indicates on the user interface of the machine that a new (2^{nd}) treatment shall be started.

The system will unlock the lids for the 2^{nd} set of ports and switch the LEDs for the 2^{nd} set of ports from red to green to indicate readiness for the setup of the 2^{nd} treatment.

After the concentrates and the bicarbonate solutions have been connected, the arterial and venous conduit systems are inserted, wherein the respective blood conduits are guided through corresponding clamps and connected to the respective sensors. The conduit (10) is connected pre-pump between the arterial blood conduit (9) to the 2^{nd} substitution port (13), which is indicated by a green LED, and whose lid is unlocked by the control system.

For priming, the patient end of the venous line is connected to the 2^{nd} waste port (15) which is indicated by a green LED, and whose lid is unlocked by the control system (not shown).

Substitution fluid from the 2^{nd} substitution port (13) is used to prime the conduit system and purge the air from it, wherein the blood pump is automatically stopped by the system as soon as the predetermined filling level is reached. The air and excess fluid flows to the 2^{nd} waste port (15) and from there to the drain.

After the respective treatment parameters like dialysis fluid composition, blood flow, dialysis fluid flow, minimum and maximum parameters, and heparin, if required, are entered, the dialyzer is coupled to the dialyzing fluid system. The system is now tested for leaks within the dialyzing fluid system. Afterwards, the dialysis machine is ready for the 2^{nd} patient to be connected for treatment.

The 2^{nd} patient is connected to the blood conduits. The lid of the waste port is closed and locked, and the blood pump is started, dialysis fluid flow is started. Treatment is started and typically lasts 3.5-4h.

### Fluid bolus during treatment

During treatment, a fluid bolus can be provided. The blood pump will be stopped, then a valve will be opened to allow flow from the 2^{nd} substitution port (13) to the blood conduit, the arterial line will be clamped upstream from the point where the substitution fluid enters the blood conduit. Now the blood pump will be started to administer a certain amount of fluid, for instance 200 ml. As the arterial line is clamped, the fluid is flowing from the 2^{nd} substitution port (13) to the blood conduit and then is being pumped to the patient's venous connection. Once the required amount is administered, the fluid valve will be closed to stop flow from the 2^{nd} substitution port (13) and the arterial conduit can be unclamped. The therapy will continue.

### End of 2^{nd} Therapy

At the end of the therapy the blood pump is stopped. The blood reconstitution can happen in two steps. In a first step, the valve for substitution fluid from the 2^{nd} substitution fluid port is opened. As there is a positive pressure from the 2^{nd} substitution port (13), the substitution fluid will flow to the blood conduit and push back the blood in the arterial blood conduit to the patient. Once this is finished, the arterial blood conduit is clamped proximal to the patient. In a 2^{nd} step, the blood pump will be started, thus using fluid form the 2^{nd} substitution port (13) to push back the blood into the venous blood conduit back to the patient. Once this is finished, the blood pump will stop and the venous blood conduit is clamped proximal to the patient. Now all the blood is returned to the patient and the patient can be disconnected from the arterial and venous blood conduit.

The blood conduit, and the dialyzer can now be removed from the dialysis machine. The lid of the 1^{st} substitution port (13) and 1^{st} waste port (15) will be closed and then locked, the LEDs for the used 2^{nd} set of ports will turn red (not shown).

The surface of the machine will be disinfected.

End of 2^{nd} Therapy.
Both port pairs have been used, the machine needs to be disinfected before any next treatment. The lids of the ports cannot be unlocked and opened before the disinfection has been performed successfully.

### BRIEF DESCRIPTION OF THE DRAWINGS

The invention is best understood from the following detailed description when read in connection with the accompanying drawings. Included in the drawings are the following figures:
FIG. 1 shows an HD machine with waste port without online fluid preparation, which represents the state of the art,
FIG. 2 shows an HDF machine with substitution port and waste port which represents the state of the art,
FIG. 3 shows a schematic of the dialysis machine according to aspects of the invention for HDF therapy machine with two pairs of ports in a 1^{st} therapy configuration,
FIG. 4 shows a schematic of the dialysis machine according to aspects of the invention for HDF therapy machine with two pairs of ports in a 2^{nd} therapy configuration,
FIG. 5 shows an HD machine with online preparation of substitution fluid, without substitution pump, which represents the state of the art and does not support HDF, but supports online priming, bolus and rinse-back,
FIG. 6 shows a schematic of the dialysis machine according to aspects of the invention for HD treatment with online preparation of substitution fluid, without substitution pump and with two port pairs in a 1^{st} therapy configuration,
FIG. 7 shows a schematic of the dialysis machine according to aspects of the invention for HD treatment with online preparation of substitution fluid, without substitution pump and with two port pairs in a 2^{nd} therapy configuration.

### DETAILED DESCRIPTION OF THE PREFERRED EMBODIMENTS

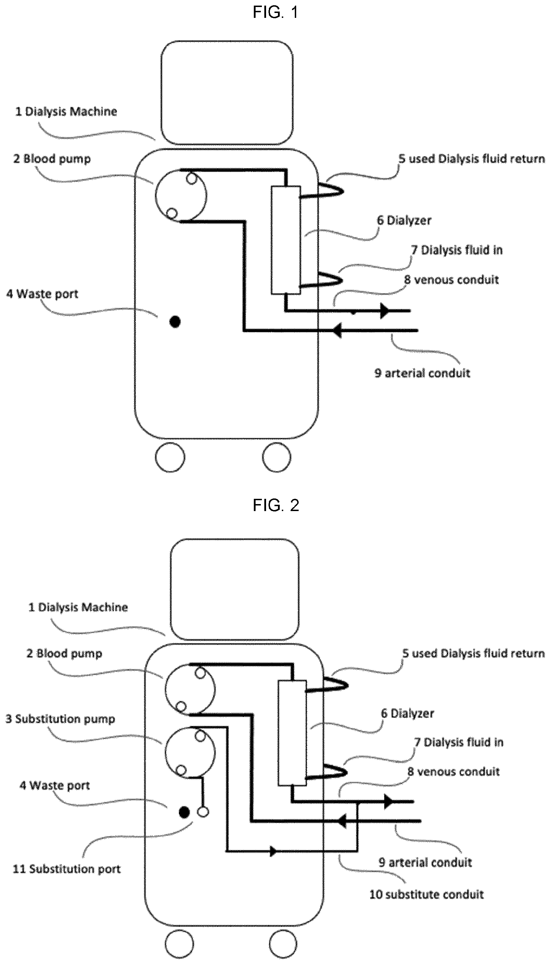

FIG. 1 illustrates a known state-of-the-art haemodialysis machine 1, comprising a blood pump 2, a dialyzer 6 with dialysis fluid inlet 7 and outlet 5 as well as the extracorporeal blood system 8 and 9 furthermore a waste port (4). All conduits are accessible for a user, usually a patient or medical staff, for operating and handling the dialysis machine 1. Dialysis fluid is conveyed via a dialysis fluid in line 7 to a dialyzer 6 arranged outside the machine casing. From there a dialysis fluid drain 5 leads to the drain. Dialysis fluid is pumped through the dialyzing fluid system. The only one waste port (4) has to be considered contaminated after the 1^{st} use. It cannot be excluded that it has been left open too long, has been touched or got in contact with body fluids or other sources of contamination.

Therefore, even though after each treatment the blood containing or contacting conduits 5, 7, 8, 9 and the dialyzer 6 are replaced, an entire cleaning and disinfection procedure is required since only one waste port is present.

FIG. 2 illustrates a known state-of-the-art haemodiafiltration machine 1 in a post dilution mode, since the substitute conduit 10 is connected to the venous conduit 8. In comparison to the HD machine according to FIG. 1, a substitution pump 3 is present in order to pump the online produced substitution fluid. Depending on the connection of the substitute conduit 10, bolus, priming or rinse-back is also possible. Phrased differently, downstream of the substitution pump 3 the substitution conduit 10 can be split into two substitution conduits, wherein one substitution conduit serves for feeding substitution fluid post-dilution, while the other substitution conduit serves for feeding substitution fluid pre-dilution, while being connected to the arterial conduit 9. Also, both dilutions can be performed at the same time. The substitution fluid pump 3 is controlled by a computing unit not shown in the Figure. Also, in the dialysis fluid in 7 a filter can be arranged and in the substitution conduit 10 another filter could be arranged. By controlling valves there can be selected or changed between pre-dilution and post-dilution and, respectively, pre-bolus administration and post-bolus administration. Moreover, there is the option of a mixed dilution in which substitution and, respectively, bolus administration is performed pre- and post-dialyzer.

The only one waste port (4) and only one substitution port (11) have to be considered contaminated after the 1^{st} use. It cannot be excluded that they have been left open too long, have been touched or got in contact with body fluids or other sources of contamination.

Therefore, even though after each treatment the blood containing or contacting conduits 5, 7, 8, 9, the dialyzer 6 and the substitution conduit 10 are replaced, an entire cleaning and disinfection procedure is required since only one waste 4 and one substitution 11 port is present.

FIG. 3 shows a schematic of the dialysis machine according to aspects of the invention for HDF therapy machine in a 1^{st} therapy configuration in a post dilution mode, since the substitute conduit 10 is connected to the venous conduit 8 post dialyzer. The dialysis machine 1 comprises a blood pump 2, a dialyzer 6 with dialysis fluid inlet 7 and outlet 5 as well as the extracorporeal blood system 8 and 9 furthermore a first waste port (14) and a 2^{nd} waste port (15) and furthermore a 1^{st} substitution port (14) and a 2^{nd} substitution port (15). All conduits are accessible for a user, usually a patient or medical staff, for operating and handling the dialysis machine 1. Dialysis fluid is conveyed via a dialysis fluid in line 7 to a dialyzer 6 arranged outside the machine casing. From there a dialysis fluid drain 5 leads to the drain. Dialysis fluid is pumped through the dialyzing fluid system. A substitution pump 3 is present in order to pump the online produced substitution fluid from the first substitution port 12. Depending on the connection of the substitute conduit 10, bolus, priming or rinse-back is also possible. Phrased differently, downstream of the substitution pump 3 the substitution conduit 10 can be split into two substitution conduits, wherein one substitution conduit serves for feeding substitution fluid post-dilution, while the other substitution conduit serves for feeding substitution fluid pre-dilution, while being connected to the arterial conduit 9. Also, both dilutions can be performed at the same time. The substitution fluid pump 3 is controlled by a computing unit not shown in the Figure. Also, in the dialysis fluid in 7 a filter can be arranged and in the substitution conduit 10 another filter could be arranged. By controlling valves, it can be selected or changed between pre-dilution and post-dilution and, respectively, pre-bolus administration and post-bolus administration. Moreover, there is the option of a mixed dilution in which substitution and, respectively, bolus administration is performed pre- and post-dialyzer.

Thus, since after each treatment the blood containing or contacting conduits 5, 7, 8, 9, the dialyzer 6 and the substitution conduit 10 are replaced, an entire cleaning and disinfection procedure is not required before a next (i.e. 2^{nd} therapy) treatment with another patient can be started, since the first waste port 14 and the first substitution port 12 is closed and the second waste 15 and second substitution port 13 are used for a 2^{nd} treatment, which are still sterile and considered non-contaminated. FIG. 4 illustrates the respective connection within a second treatment without intermediate cleaning and disinfection procedure.

FIG. 5 illustrates a known state-of-the-art PBR haemodialysis machine 1, comprising a blood pump 2, a dialyzer 6 with dialysis fluid inlet 7 and outlet 5 as well as the extracorporeal blood system 8 and 9 furthermore a waste port (4) and a substitution port (11). All conduits are accessible for a user, usually a patient or medical staff, for operating and handling the dialysis machine 1. Dialysis fluid is conveyed via a dialysis fluid in line 7 to a dialyzer 6 arranged outside the machine casing. From there a dialysis fluid conduit 5 leads to the drain. Dialysis fluid is pumped through the dialyzing fluid system. In contrast to the dialysis machine according to FIG. 1, the substitution solution is prepared online and enters the system via the substitution port 11.

By controlling valves, it can be selected or changed between pre-dilution and post-dilution and, respectively, pre-bolus administration and post-bolus administration of substitution solution. Moreover, there is the option of a mixed dilution in which substitution and, respectively, bolus administration is performed pre and post, i.e., when the substitution conduit 10 is also connected to the venous conduit 8, pre or post dialyzer.

The only one waste port (4) and only one substitution port (11) have to be considered contaminated after the 1^{st} use. It cannot be excluded that they have been left open too long, have been touched or got in contact with body fluids or other sources of contamination.

Therefore, even though after each treatment the blood containing or contacting conduits 5, 7, 8, 9 and the dialyzer 6 are replaced, an entire cleaning and disinfection procedure is required since only one waste port 4 and one substitution port 11 is present.

FIG. 6 shows a schematic of the dialysis machine according to aspects of the invention for HD therapy machine in a 1^{st} therapy configuration in a pre-dilution mode since the substitute conduit 10 is connected to the arterial conduit 9. The dialysis machine 1 comprises a blood pump 2, a dialyzer 6 with dialysis fluid inlet 7 and outlet 5 as well as the extracorporeal blood system 8 and 9 furthermore a first waste port (14) and a 2^{nd} waste port (15) and furthermore a 1^{st} substitution port (14) and a 2^{nd} substitution port (15). All conduits are accessible for a user, usually a patient or medical staff, for operating and handling the dialysis machine 1. Dialysis fluid is conveyed via a dialysis fluid in line 7 to a dialyzer 6 arranged outside the machine casing. From there a dialysis fluid conduit 5 leads to the drain. Dialysis fluid is pumped through the dialyzing fluid system.

The substitution solution is prepared online and taken from the first substitution port 12. Depending on the connection of the substitute conduit 10, bolus, priming or rinse-back is also possible. Phrased differently, downstream of the first substitution port 12 the substitution conduit 10 can be split into two substitution conduits, wherein one substitution conduit serves for feeding substitution fluid post-dilution, while being connected to the venous conduit 8, whereas the other substitution conduit serves for feeding substitution fluid pre-dilution, while being connected to the arterial conduit 9. Also, both dilutions can be performed at the same time. Also, in the dialysis fluid in 7 a filter can be arranged and in the substitution conduit 10 another filter could be arranged. By controlling valves it can be selected or changed between pre-dilution and post-dilution and, respectively, pre-bolus administration and post-bolus administration. Moreover, there is the option of a mixed dilution in which substitution and, respectively, bolus administration is performed pre and post.

Thus, since after each treatment the blood containing or contacting conduits 5, 7, 8, 9, the dialyzer 6 and the substitution conduit 10 are replaced, an entire cleaning and disinfection procedure is not required before a next (i.e. 2^{nd} therapy) treatment with another patient can be started, since the first waste port 14 and the first substitution port 12 is closed and the second waste 15 and second substitution port 13 are used, which are still sterile and considered non-contaminated. FIG. 7 illustrates the respective connection within a second treatment without intermediate cleaning and disinfection procedure.

## Claims

1. A dialysis machine (1) comprising:
an extracorporeal blood conduction system and a dialyzing fluid system, wherein the extracorporeal blood conduction system is in communication with the dialyzing fluid system via at least one dialyzer (6);
further comprising at least one blood pump (2) which can convey fluid in the extracorporeal blood conduction system,
wherein the extracorporeal blood conduction system comprises at least one venous and one arterial conduit (9), the arterial conduit (9) leading up to the dialyzer (6) and the venous conduit (8) leading away from the dialyzer (6) and the respective other end of the venous and arterial conduit (9) being intended to be connected to a patient,
further comprising at least one substitute conduit (10) for supplying fresh substitution fluid either into the arterial conduit (9) or into the venous conduit (8) or into both the arterial and the venous conduit (8), **characterized in that** at least two hydraulic connections (12, 13) are present at the dialysis machine (1), to each of which one substitute conduit (10) is connectable for supplying fresh substitution fluid, wherein at least two further hydraulic connections (14, 15) are present, wherein the at least two further hydraulic connections (14, 15) are waste ports (14, 15) that, in use, convey used fluid to the drain

2. The dialysis machine (1) according to claim 1, wherein at least one substitution pump (3) is present which can convey dialysis fluid in each of the substitute conduits (10).

3. The dialysis machine (1) according to claims 1 or 2, wherein the hydraulic connections (12, 13) are each equipped with a connection sensor, preferably wherein the hydraulic connections (12, 13) are substitution ports (12, 13).

4. The dialysis machine (1) according to any one of claims 1-3, wherein the at least two hydraulic connections (12, 13) are each provided with a respective status indicator.

5. The dialysis machine (1) according to any one of claims 1-4, wherein the at least two hydraulic connections (12, 13) are Luer connectors.

6. The dialysis machine (1) according to any one of claims 1-5, wherein at least one filtration device is present.

7. The dialysis machine (1) according to any one of claims 1-6, wherein the at least one blood pump (2) and/or the at least one substitution pump (9) is a peristaltic pump and wherein the number of waste ports (14, 15) is equal to the number of substitution ports (12, 13).

8. The dialysis machine (1) according to any one of claims 3-7, wherein each substitution port (12, 13) and each waste port (14, 15) are grouped together in order to form a port-pair and wherein at least two port-pairs are equipped with separate covers, preferably wherein the connection sensors detect an opening of one of the covers, while a control mechanism locks the cover of the remaining hydraulic connections.

9. The dialysis machine (1) according to claim 8, wherein the control mechanism further controls the status of the substitution ports (12, 13) and the waste ports (14, 15) and the dialysis machine (1).

10. The dialysis machine (1) according to any of claims 8 or 9-, wherein at least one valve is present which controls the flow of fresh substitution fluid from each of the hydraulic connections (12, 13) or of used substitution fluid to the hydraulic connections (14, 15), wherein the at least one valve is controlled by the control mechanism.

11. The dialysis machine (1) according to claims 1-10, wherein the substitution ports (12, 13) and waste ports (14, 15) are covered with lids, while not in use.

## Patentansprüche

1. Dialysegerät (1), umfassend:
ein extrakorporales Blutleitungssystem und ein Dialyseflüssigkeitssystem, wobei das extrakorporale Blutleitungssystem über mindestens einen Dialysator (6) mit dem Dialyseflüssigkeitssystem in Verbindung steht;
ferner umfassend mindestens eine Blutpumpe (2), die in der Lage ist, Flüssigkeit in dem extrakorporalen Blutleitungssystem zu befördern,
wobei das extrakorporale Blutleitungssystem mindestens eine venöse und eine arterielle Leitung (9) umfasst, die arterielle Leitung (9) zum Dialysator (6) führt, die venöse Leitung (8) vom Dialysator (6) fort führt und das jeweils andere Ende der venösen bzw. arteriellen Leitung (9) dazu vorgesehen ist, mit einem Patienten verbunden zu werden, ferner umfassend mindestens eine Ersatzleitung (10) zum Zuführen von frischer Ersatzflüssigkeit entweder in die arterielle Leitung (9) oder in die venöse Leitung (8) oder in sowohl die arterielle als auch die venöse Leitung (8), **dadurch gekennzeichnet, dass**
mindestens zwei hydraulische Verbindungen (12, 13) am Dialysegerät (1) vorhanden sind, an die jeweils eine Ersatzleitung (10) zum Zuführen von frischer Ersatzflüssigkeit anschließbar ist, wobei mindestens zwei weitere hydraulische Verbindungen (14, 15) vorhanden sind, wobei die mindestens zwei weiteren hydraulischen Verbindungen (14, 15) Rückstandsanschlüsse (14, 15) sind, die, wenn sie in Gebrauch sind, verbrauchte Ersatzflüssigkeit zum Abfluss befördern.

2. Dialysegerät (1) nach Anspruch 1, wobei mindestens eine Ersatzpumpe (3) vorhanden ist, die Dialyseflüssigkeit in jeder der Ersatzleitungen (10) befördern kann.

3. Dialysegerät (1) nach Anspruch 1 oder 2, wobei die hydraulischen Verbindungen (12, 13) jeweils mit einem Verbindungssensor ausgerüstet sind, wobei die hydraulischen Verbindungen (12, 13) bevorzugt Ersatzanschlüsse (12, 13) sind.

4. Dialysegerät (1) nach einem der Ansprüche 1 bis 3, wobei die mindestens zwei hydraulischen Verbindungen (12, 13) jeweils mit einer entsprechenden Statusanzeige versehen sind.

5. Dialysegerät (1) nach einem der Ansprüche 1 bis 4, wobei die mindestens zwei hydraulischen Verbindungen (12, 13) Luer-Verbinder sind.

6. Dialysegerät (1) nach einem der Ansprüche 1 bis 5, wobei mindestens eine Filtrationseinrichtung vorhanden ist.

7. Dialysegerät (1) nach einem der Ansprüche 1 bis 6, wobei die mindestens eine Blutpumpe (2) und/oder die mindestens eine Ersatzpumpe (9) eine Peristaltikpumpe ist und wobei die Anzahl der Rückstandsanschlüsse (14, 15) gleich der Anzahl der Ersatzanschlüsse (12, 13) ist.

8. Dialysegerät (1) nach einem der Ansprüche 3 bis 7, wobei jeder Ersatzanschluss (12, 13) und jeder Rückstandsanschluss (14, 15) gruppiert sind, um ein Anschlusspaar zu bilden, und wobei mindestens zwei Anschlusspaare mit separaten Abdeckungen ausgerüstet sind, vorzugsweise wobei die Verbindungssensoren das Öffnen einer der Abdeckungen erfassen, während ein Steuerungsmechanismus die Abdeckung der verbleibenden hydraulischen Verbindungen arretiert.

9. Dialysegerät (1) nach Anspruch 8, wobei der Steuerungsmechanismus ferner den Status der Ersatzanschlüsse (12, 13) und der Rückstandsanschlüsse (14, 15) und das Dialysegerät (1) steuert.

10. Dialysegerät (1) nach einem der Ansprüche 8 oder 9, wobei mindestens ein Ventil vorhanden ist, das den Fluss von frischer Ersatzflüssigkeit aus jeder der hydraulischen Verbindungen (12, 13) oder von verbrauchter Ersatzflüssigkeit zu den hydraulischen Verbindungen (14, 15) steuert, wobei das mindestens eine Ventil durch den Steuerungsmechanismus gesteuert wird.

11. Dialysegerät (1) nach Anspruch 1 bis 10, wobei die Ersatzanschlüsse (12, 13) und die Rückstandsanschlüsse (14, 15) mit Deckeln abgedeckt sind, während sie nicht in Gebrauch sind.

## Revendications

1. Un appareil de dialyse (1) comprenant:
un système de conduction sanguine extracorporel et un système de fluide de dialyse, dans lequel le système de conduction sanguine extracorporel est en communication avec le système de fluide de dialyse via au moins un dialyseur (6);
comprenant en outre au moins une pompe à sang (2) pouvant transporter du fluide dans le système de conduction sanguine extracorporel,
dans lequel le système de conduction sanguine extracorporel comprend au moins un conduit veineux et un conduit artériel (9), le conduit artériel (9) menant au dialyseur (6) et le conduit veineux (8) s'éloignant du dialyseur (6) et l'autre extrémité respective du conduit veineux et du conduit artériel (9) étant destinée à être connectée à un patient,
comprenant en outre au moins un conduit de substitution (10) pour permettre l'alimentation en fluide de substitution frais soit du conduit artériel (9), soit du conduit veineux (8), soit à la fois du conduit artériel et du conduit veineux (8), **caractérisé en ce qu'**au moins deux raccords hydrauliques (12, 13) sont présents au niveau de la machine de dialyse (1), chacun d'eux étant raccordé à un conduit de substitution (10) pour l'alimentation en fluide de substitution frais, dans lequel au moins deux autres raccords hydrauliques (14,15) sont présents, dans lequel au moins deux autres raccords hydrauliques (14, 15) sont des orifices d'évacuation (14, 15) qui, sont utiles, à transporter le fluide de substitution usagé vers le drain.

2. L'appareil de dialyse (1) selon la revendication 1, dans lequel au moins une pompe de substitution (3) est présente qui peut transporter le fluide de dialyse dans chacun des conduits de substitution (10).

3. L'appareil de dialyse (1) selon les revendications 1 ou 2, dans lequel les raccords hydrauliques (12, 13) sont chacun équipés d'un capteur de raccord, de préférence dans lequel les raccords hydrauliques (12, 13) sont des orifices de substitution (12, 13).

4. L'appareil de dialyse (1) selon l'une quelconque des revendications 1-3, dans lequel au moins deux raccords hydrauliques (12, 13) sont chacun pourvu d'un indicateur de statut respectif.

5. L'appareil de dialyse (1) selon l'une quelconque des revendications 1-4, dans lequel au moins deux raccords hydrauliques (12, 13) sont des raccords Luer.

6. L'appareil de dialyse (1) selon l'une quelconque des revendications 1-5, dans lequel au moins un dispositif de filtration est présent.

7. L'appareil de dialyse (1) selon l'une quelconque des revendications 1-6, dans lequel au moins une pompe à sang (2) et/ou au moins une pompe de substitution (9) est une pompe péristaltique et dans lequel le nombre d'orifices d'évacuation (14, 15) est égal au nombre d'orifices de substitution (12, 13).

8. L'appareil de dialyse (1) selon l'une quelconque des revendications 3-7, dans lequel chaque orifice de substitution (12, 13) et chaque orifice d'évacuation (14, 15) sont regroupés afin de former une paire d'orifices et dans lequel au moins deux paires d'orifices sont équipées de couvercles séparés, de préférence dans lequel les capteurs de raccords détectent une ouverture de l'un des couvercles, tandis qu'un mécanisme de contrôle verrouille les couvercles des raccords hydrauliques restants.

9. L'appareil de dialyse (1) selon la revendication 8, dans lequel le mécanisme de contrôle commande en outre le statut des orifices de substitution (12, 13) et des orifices d'évacuation (14, 15) et l'appareil de dialyse (1).

10. L'appareil de dialyse (1) selon l'une quelconque des revendications 8 ou 9, dans lequel au moins une vanne est présente qui contrôle l'écoulement du fluide de substitution frais de chacun des raccords hydrauliques (12, 13) ou du fluide de substitution usagé vers les raccords hydraulique (14, 15), dans lequel au moins une vanne est commandée par le mécanisme de contrôle.

11. L'appareil de dialyse (1) selon les revendications 1-10, dans lequel les orifices de substitution (12, 13) et les orifices d'évacuation (14, 15) sont recouverts de couvercles lorsqu'ils ne sont pas utilisés.
